# EUROPÄISCHE PATENTANMELDUNG

(11) **EP 1 655 680 A1**
(43) Veröffentlichungstag der Anmeldung: **10.05.2006**
(21) Anmeldenummer: 04026023.4
(22) Anmeldetag: 03.11.2004
(51) Int. Cl.: G06F 19/00

(54) **Elektronische Verordnung**

(71) Anmelder: Hellmann, Gunther, Dr., 91054 Erlangen (DE)
(72) Erfinder: Hellmann, Gunther, Dr., 91054 Erlangen (DE)

(57) **Zusammenfassung**

Elektronische Verordnung (z.B. elektronisches Rezept) besteht aus mehreren Verordnungseinheiten (z.B. Medikamente) und ggf. zusätzlichen Informationseinheiten sowie einer elektronischen Signatur, die über den zuvor genannten Teilen ermittelt wird. Dabei wird das Hash-Verfahren der Signatur einzeln auf die zuvor genannten Bestandteile angewendet und anschließend die Folge aus den zusammengesetzten Hash-Werten dem zweiten Schritt der elektronischen Signatur zugeführt. Die elektronische Verordnung wird auf einem Speichermedium (z.B : Gesundheitskarte) abgelegt oder geändert, wobei auch die Ablage auf Servern möglich ist, deren Ablageadresse über die Gesundheitskarte referenziert wird. Im Falle einer Teilbelieferung der elektronischen Verordnung wird die belieferte Verordnungseinheit durch deren Hash-Wert ersetzt.

Es ergeben sich weitere Formen der elektronischen Verordnung:
1. Entsprechend der Anzahl der Verordnungseinheiten sind zusätzliche Informationseinheiten vorzusehen, die separat von der elektronischen Verordnung abgelegt werden. Der zugehörige Hash-Wert, berechnet über der Folge der Hash-Werte dieser Informationseinheiten wird in der elektronischen Verordnung integriert.
2. Es wird nur eine Informationseinheit für die gesamte Verordnung vorgesehen, die separat von der elektronischen Verordnung abgelegt wird. Der zugehörige Hash-Wert wird in der elektronischen Verordnung integriert.
3. Zu jeder belieferten Verordnungseinheit wird eine Kopie deren als separate Informationseinheit abgelegt, die im falle der Signaturprüfung mit Signaturkarte mit der belieferten Verordnungseinheit vergleichen wird.
   Zusätzlich:
4. Zu jeder separat abgelegten Informationseinheit wird zusätzlich geprüft, ob der in der elektronischen Verordnung enthaltene und assoziierte Informationsblock oder die assoziierte Verordnungseinheit übereinstimmt.

## Beschreibung

### Stand der Technik:

Die Elektronische Verordnung nach der Europäischen Patentanmeldung 04009217-3 vom 19.04.2004 ist bekannt.

### Problem:

Der im Patentanspruch 1 angegebenen Erfindung liegt das Problem zugrunde, die Speichermenge zu reduzieren.

Der im Patentanspruch 2 angegebenen Erfindung liegt das Problem zugrunde, die Speichermenge der separat abgelegten Informationsblöcke zu reduzieren.

Der im Patentanspruch 3 angegebenen Erfindung liegt das Problem zugrunde, die Teilbelieferung für Patentanspruch 2 sicher zu machen. Durch Duplizieren des elektronischen Orginalrezeptes könnte diese missbräuchlich wieder eingelöst werden.

Der im Patentanspruch 4 angegebenen Erfindung liegt das Problem zugrunde, mögliche Veränderungen an der elektronischen Verordnung zusätzlich zu erkennen.

### Lösung:

Dieses Problem wird durch die im Patentanspruch 1, 2, 3 und 4 aufgeführten Merkmale gelöst.

### Erreichte Vorteile:

Die mit der Erfindung in Anspruch 1 erzielten Vorteile bestehen insbesondere darin, dass statt mehreren Hash-Werten, die zu den separat abgelegten Informationsblöcken gehören, nun nur noch ein Hash-Wert gespeichert werden muss. Damit verbleibt mehr Speicherplatz für andere Daten auf der Gesundheitskarte. Die Patienteneinsicht ohne Signaturkarte ist weiterhin gegeben.

Die mit der Erfindung in Anspruch 2 erzielten Vorteile bestehen insbesondere darin, dass anstelle von mehreren separat abgelegten Informationsblöcken initial nur ein Block gespeichert werden muss.

Die mit der Erfindung in Anspruch 3 erzielten Vorteile bestehen insbesondere darin, dass eine durchgeführte Teilbelieferung eines oder mehrerer Verordnungseinheiten durch einen Health Professional erkannt wird. Es wird ausgeschlossen, dass ein dupliziertes Orginalrezept missbräuchlich nochmals eingelöst wird. Die Patienteneinsicht in das Rezept ohne Signaturkarte ist weiterhin möglich.

Die mit der Erfindung in Anspruch 4 erzielten Vorteile bestehen insbesondere darin, dass durch den Vergleich mögliche Modifikationen an der elektronische Verordnung erkannt werden und somit die Sicherheit erhöht wird.

## Patentansprüche

1. Elektronische Verordnung nach Anspruch 1 und 3 der Europäischen Patentanmeldung 04009217-3 vom 19.04.2004
**dadurch gekennzeichnet,**
**dass** zu jeder Verordnungseinheit eine Informationseinheit,
• die separat von der elektronischen Verordnung abgelegt ist,
• die bei einer Teilbelieferung der zugehörigen "belieferten" Verordnungseinheit durch ihren Hash-Wert ersetzt wird,
• das der Hash-Wert über der Folge der von den Informationsblöcken berechneten Hash-Werte oder den ersetzten Hash-Werten in der elektronischen Verordnung als ein Informationsblock gespeichert ist,
• die bei Signaturprüfung der elektronischen Verordnung mit Signaturkarte herangezogen wird und
• deren Hash-Wert aus der elektronischen Verordnung bei Signaturprüfung ohne Signaturkarte herangezogen wird,
assoziiert ist.

2. Elektronische Verordnung nach Anspruch 1 und 3 der Europäischen Patentanmeldung 04009217-3 vom 19.04.2004
**dadurch gekennzeichnet,**
**dass** zu der elektronischen Verordnung eine Informationseinheit,
• die separat von der elektronischen Verordnung abgelegt ist,
• das der Hash-Wert in der elektronischen Verordnung als ein Informationsblock gespeichert ist,
assoziiert ist.

3. Elektronische Verordnung nach Anspruch 1 und 3 der Europäischen Patentanmeldung 04009217-3 vom 19.04.2004 und obigem Anspruch 2
**dadurch gekennzeichnet,**
**dass** zu jeder belieferten Verordnungseinheit eine Informationseinheit,
• die separat von der elektronischen Verordnung abgelegt ist,
• die eine Kopie der belieferten Verordnungseinheit ist,
• die bei Signaturprüfung der elektronischen Verordnung mit Signaturkarte herangezogen wird und
• deren Hash-Wert aus der elektronischen Verordnung bei Signaturprüfung ohne Signaturkarte herangezogen wird,
assoziiert ist.

4. Elektronische Verordnung nach Anspruch 2 der Europäischen Patentanmeldung 04009217-3 vom 19.04.2004 oder obigem Anspruch 1 oder obigem Anspruch 2 oder obigem Anspruch 3
**dadurch gekennzeichnet,**
**dass** jede Informationseinheit,
• die separat von der elektronischen Verordnung abgelegt ist,
• bei Signaturprüfung der elektronischen Verordnung mit Signaturkarte mit der in der elektronischen Verordnung assoziierten Verordnungseinheit oder assoziierten Informationseinheit vergleichen wird und
• bei Signaturprüfung ohne Signaturkarte nicht vergleichen wird.
